# EUROPÄISCHE PATENTSCHRIFT

(11) **EP 1 412 528 B1**
(45) Veröffentlichungstag und Bekanntmachung des Hinweises auf die Patenterteilung: **02.03.2011**
(21) Anmeldenummer: 02754417.0
(22) Anmeldetag: 23.07.2002
(51) Int. Cl.: C12Q 1/68

(54) **BIOSENSOR UND VERFAHREN ZUM ERFASSEN VON MAKROMOLEKULAREN BIOPOLYMEREN MITTELS MINDESTENS EINER EINHEIT ZUM IMMOBILISIEREN VON MAKROMOLEKULAREN BIOPOLYMEREN**
BIOSENSOR AND METHOD FOR DETECTING MACROMOLECULAR BIOPOLYMERS BY MEANS OF AT LEAST ONE UNIT FOR IMMOBILIZING MACROMOLECULAR BIOPOLYMERS
BIOCAPTEUR ET PROCEDE DE DETECTION DE BIOPOLYMERES MACROMOLECULAIRES AU MOYEN D'AU MOINS UNE UNITE POUR IMMOBILISER DES BIOPOLYMERES MACROMOLECULAIRES

(30) Priorität: 31.07.2001 DE 10137342
(43) Veröffentlichungstag der Anmeldung: 28.04.2004
(73) Patentinhaber: SIEMENS AKTIENGESELLSCHAFT, 80333 München (DE)
(72) Erfinder: HOFMANN, Franz, 80995 München (DE); LUYKEN, Richard, Johannes, 81825 Munchen (DE); SCHINDLER-BAUER, Petra Theresia, D- 85591 Vaterstetten (DE)
(74) Vertreter: Viering, Jentschura & Partner
(86) Internationale Anmeldenummer: PCT/DE2002/002706
(87) Internationale Veröffentlichungsnummer: WO 2003/014695

(56) Entgegenhaltungen:
- WO-A-88/08875
- WO-A-99/32662
- DE-A- 19 736 641
- US-A- 5 837 465
- US-A- 6 017 696
- US-B1- 6 197 503
- US-B1- 6 333 200
- HSUEH Y-T ET AL: "DNA quantification with an electrochemiluminescence microcell" SENSORS AND ACTUATORS B, ELSEVIER SEQUOIA S.A., LAUSANNE, CH, Bd. 49, Nr. 1-2, 25. Juni 1998 (1998-06-25), Seiten 1-4, XP004141429 ISSN: 0925-4005

## Beschreibung

Die Offenbarung betrifft einen Biosensor und ein Verfahren zum Erfassen von makromolekularen Biopolymeren mittels mindestens einer Einheit zum Immobilisieren von makromolekularen Biopolymeren.

Aus [1] bis [4] sind Verfahren zum Erfassen von DNA-Molekülen bekannt, bei denen zur Erfassung Biosensoren eingesetzt werden, die auf Elektrodenanordnungen basieren.

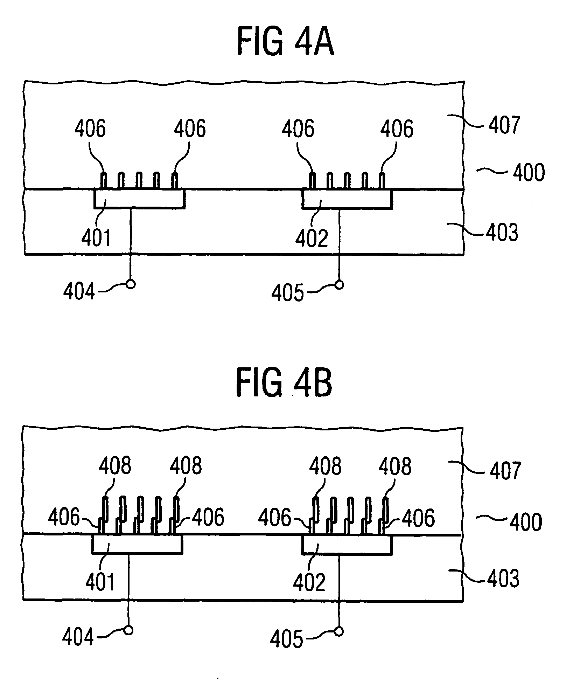

**Fig.4a** und **Fig.4b** zeigen einen solchen Sensor, wie er in [1] und [4] beschrieben ist. Der Sensor 400 weist zwei Elektroden 401, 402 aus Gold auf, die in einer Isolatorschicht 403 aus Isolatormaterial eingebettet sind. An die Elektroden 401, 402 sind Elektroden-Anschlüsse 404, 405 angeschlossen, an denen das an der Elektrode 401, 402 anliegende elektrische Potential zugeführt werden kann. Die Elektroden 401, 402 sind als Planarelektroden angeordnet. Auf jeder Elektrode 401, 402 sind DNA-Sondenmoleküle 406 immobilisiert (vgl. **Fig.4a****).** Die Immobilisierung erfolgt gemäß der sogenannten Gold-Schwefel-Kopplung. Auf den Elektroden 401, 402 ist der zu untersuchende Analyt 407, aufgebracht. Der Analyt kann dabei beispielsweise eine elektrolytische Lösung verschiedener DNA-Moleküle sein.

Sind in dem Analyt 407 DNA-Stränge 408 mit einer Sequenz enthalten, die zu der Sequenz der DNA-Sondenmoleküle 406 komplementär ist, so hybridisieren diese DNA-Stränge 408 mit den DNA-Sondenmolekülen 406 **(vgl.** **Fig.4b****).**

Eine Hybridisierung eines DNA-Sondenmoleküls 406 und eines DNA-Strangs 408 findet nur dann statt, wenn die Sequenzen des jeweiligen DNA-Sondenmoleküls 406 und des entsprechenden DNA-Strangs 408 zueinander komplementär sind. Ist dies nicht der Fall, so findet keine Hybridisierung statt. Somit ist ein DNA-Sondenmolekül einer vorgegebenen Sequenz jeweils nur in der Lage einen bestimmten, nämlich den DNA-Strang mit jeweils komplementärer Sequenz zu binden, d.h. mit ihm zu hybridisieren.

Findet eine Hybridisierung statt, so verändert sich, wie aus **Fig.4b** ersichtlich, neben anderen elektrischen Parametern auch die Kapazität zwischen den Elektroden. Diese Änderung der Kapazität kann als Messgröße für die Erfassung von DNA-Molekülen herangezogen werden.

Weiterhin ist ein Reduktions-/Oxidations-Recycling-Verfahren zum Erfassen makromolekularer Biopolymere aus [1], [2] und [3] bekannt. Bei diesem befindet sich an den beispielweise zu erfassenden Proteinen eine redoxaktive Markierung. Nach der Bindung der zu erfassenden Proteine an Fängermoleküle wird durch diese Markierung ein Zyklus aus Oxidation und Reduktion von geeigneten Molekülen ausgelöst, der zu einem für den Nachweis der Proteine verwendeten elektrischen Kreisstrom führt.

Hauptsächlich werden zum Nachweis von makromolekularen Biopolymeren wie DNA-Molekülen jedoch optische Verfahren eingesetzt, die auf der Verwendung von Fluoreszenz-Farbstoffen beruhen. So ist z.B. aus [5] eine Vorgehensweise für die Untersuchung des Elektrolyts auf die Existenz eines DNA-Strangs mit vorgegebener Sequenz bekannt. Bei dieser Vorgehensweise werden die DNA-Stränge der gewünschten Sequenz mit einem Fluoreszenz-Farbstoff markiert und deren Existenz wird anhand der Fluoreszenzeigenschaften der markierten Moleküle bestimmt. Hierzu wird Licht beispielsweise im sichtbaren oder ultravioletten Wellenlängenbereich auf den Elektrolyten gestrahlt und es wird das von dem Analyten, insbesondere von dem nachzuweisenden markierten DNA-Strang, emittierte Licht erfasst. Aufgrund des Fluoreszenzverhaltens, d.h. insbesondere aufgrund der erfassten, emittierten Lichtstrahlen wird bestimmt, ob der nachzuweisende DNA-Strang mit der entsprechend vorgegebenen Sequenz in dem Analyten enthalten ist oder nicht.

Diese Vorgehensweise ist sehr aufwendig, da eine sehr genaue Kenntnis über das Fluoreszenzverhalten des entsprechenden Markermoleküls am DNA-Strang erforderlich ist und darüber hinaus eine Markierungsreaktion der DNA-Stränge vor Beginn des Verfahrens notwendig ist. Ferner ist eine sehr genaue Justierung des Erfassungsmittels zum Erfassen der emittierten Lichtstrahlen erforderlich, damit diese Lichtstrahlen überhaupt erfasst werden können.

Generell sind die auf Basis von Fluoreszenz-Farbstoffen arbeitenden Erfassungs- und Nachweisverfahren insofern nachteilig, dass die Fluoreszenzstrahlung durch ein externes Spektrometer nachgewiesen wird. Diese sind teuer und aufwändig im Betrieb.

Der Offenbarung liegt das Problem zugrunde, ein alternatives Verfahren und eine Vorrichtung für die Erfassung von makromolekularen Biopolymeren bereitzustellen.

Das Problem wird durch das Verfahren und den Biosensor mit den Merkmalen gemäß den unabhängigen Patentansprüchen gelöst.

Bei diesem Verfahren zum Erfassen von makromolekularen Biopolymeren wird mindestens eine Einheit zum Immobilisieren von makromolekularen Biopolymeren eingesetzt, welche in einem Substrat integriert ist oder auf einem Substrat aufgebracht ist.

Bei dem Verfahren wird die mindestens eine Einheit zum Immobilisieren von makromolekularen Biopolymeren mit Fängermolekülen versehen, wobei die Fängermoleküle makromolekulare Biopolymere binden können. Dann wird eine Probe mit der mindestens einen Einheit zum Immobilisieren von makromolekularen Biopolymeren in Kontakt gebracht. Dabei kann die Probe die zu erfassenden makromolekularen Biopolymere enthalten. In der Probe enthaltene makromolekulare Biopolymere werden an den Fängermolekülen gebunden. Dann wird bei dem Verfahren mittels einer Markierung die Erzeugung eines Chemilumineszenzsignals angeregt. Dieses Chemilumineszenzsignal wird von einer Erfassungseinheit erfasst, die als in dem Substrat integrierte Schaltung ausgestaltet ist. Dadurch werden die makromolekularen Biopolymere erfasst.

Einfach ausgedrückt beruht das vorliegende Verfahren auf der Erkenntnis, dass eine "on-Chip"-Detektion unter Verwendung von Chemilumineszenz-Markierungen bzw. - Strahlung mehrere Vorteile bietet. Erstens bietet sie den Vorteil, dass das gesamte Chemilumineszenzsignal zur Erfassung der makromolekularen Biopolymere herangezogen werden kann, und dabei - im Gegensatz zu auf Fluoreszenz basierten Nachweisverfahren - die emittierte Strahlung nicht von der Anregungsstrahlung abgetrennt werden muss. Zweitens kann durch die Signalerfassung und ggf. die weitere nachfolgende Signalverarbeitung auf externe Erfassungseinheiten wie Spektrometer verzichtet werden. Dies ermöglicht eine deutliche Vereinfachung und Verkleinerung des apparativen Aufbaus. Drittens erlaubt die "on-Chip"-Signaldetektion eine ortsaufgelöste Messung.

Der hier offenbarte Biosensor zum Erfassen von makromolekularen Biopolymeren weist mindestens eine Einheit zum Immobilisieren von makromolekularen Biopolymeren, welche in einem Substrat integriert ist oder auf dem Substrat aufgebracht ist, sowie eine Erfassungseinheit auf. Bei dem Biosensor ist die mindestens eine Einheit zum Immobilisieren von makromolekularen Biopolymeren mit Fängermolekülen versehen. Dabei können die Fängermoleküle makromolekulare Biopolymere binden und weisen eine Markierung auf, die ein Chemilumineszenzsignal erzeugen kann. Bei dem Biosensor ist ferner die Erfassungseinheit als eine in dem Substrat integrierte Schaltung ausgestaltet. Sie ist ferner derart ausgestaltet, dass sie makromolekulare Biopolymere, die an die Fängermoleküle gebunden haben, mittels des von der Markierung ausgesandten Signals erfasst.

In einer Ausgestaltung des Biosensors weist die Erfassungseinheit zur Erfassung des optischen Chemilumineszenzsignals mindestens eine Photodiode, CCD-Kamera oder CMOS-Kamera auf.

In einer anderen Ausführungsform weist der Biosensors mehrere Einheiten zum Immobilisieren von makromolekularen Biopolymeren in einer regelmäßigen Anordnung (einem Array) auf. Vorzugsweise ist bei dem Sensor die zumindest eine Einheit zum Immobilisieren oder die regelmäßige Anordnung der Einheiten auf der Photodiode, der CMOS-Kamera oder der CCD-Kamera aufgebracht, d.h. oberhalb dieser Erfassungseinheiten. Wird eine regelmäßige Anordnung der Erfassungseinheiten verwendet, z.B. Photodioden-Felder oder -Arrays, können eine oder mehrere Einheiten zum Immobilisieren auf jeder Erfassungseinheit, z.B. jeder Photodiode, aufgebracht sein.

Bei dem hier beschriebenen Verfahren wird mit Hilfe der Markierung ein Chemilumineszenzsignal erzeugt. Als Markierung können alle Markierungen eingesetzt werden, die durch eine chemische Reaktion mittelbar oder unmittelbar die Emission eines optischen Signals bewirken.

Ein Beispiel für eine Markierung, die von sich aus (d.h. unmittelbar im vorliegenden Sinne) die Erzeugung von Chemilumineszenzstrahlung bewirken kann, ist die Meerrettich-Peroxidase, die in der Anwesenheit von Wasserstoffperoxid (H₂O₂) die Oxidation von cyclischen Diacylhydraziden wie Luminol katalysiert. Bei dieser chemischen Umsetzung wird ein Reaktionsprodukt in einem angeregten Zustand gebildet, der durch Lichtemission in den Grundzustand übergeht. Diese Lichtemission kann durch weitere chemische Reaktionspartner verstärkt werden, im Falle des Peroxidase-Luminol-Systems z.B. durch 4-Iodphenol. Ein weiteres Beispiel ist die Luciferase aus *Photinus pyralis,* die in Gegenwart von ATP und Sauerstoff die Umwandlung von Luciferin in oxidiertes Luciferin unter Lichtemission katalysiert. Ein anderes Beispiel ist alkalische Phosphatase, die geeignete 1,2-Dioxetane als Substrate verwendet; vgl. [6]. Eine solche Markierung kann, falls gewünscht, direkt mit einem der beiden Bindungspartner (Fängermolekül oder zu erfassendes Biopolymer) verknüpft werden.

Es ist jedoch andererseits auch möglich, als Markierung eine chemische Verbindung zu verwenden, die selbst keine Chemilumineszenz-Reaktion auslösen kann, die jedoch spezifische Bindungsaffinität zu einem Bindungspartner hat, der seinerseits z.B. mit einer Enzym wie der Meerrettich-Peroxidase gekoppelt ist. Eine solche Markierung ist z.B. Biotin. Dieses besitzt eine hohe Bindungsaffinität zu dem Protein Streptavidin. Wird Streptavidin z.B. mit der oben genannten Meerrettich-Peroxidase gekoppelt, ist dieses Reagenz in der Lage, einerseits an Biotin, das als Markierung z.B. in ein nachzuweisende Biopolymer eingebaut worden ist, zu binden, und andererseits ein Chemilumineszenz-Reaktion auszulösen. Daraus wird ersichtlich, dass als Markierung/Markierungskomponente der Fängermoleküle oder zu erfassenden Biopolymere vorliegend auch Verbindungen wie Biotin, Avidin oder Digoxigenin eingesetzt werden können. Die Verwendung dieser mittelbar arbeitenden Markierungen kann von Vorteil sein, da sie bildlich gesprochen, am Anfang einer Signalverstärkungskaskade stehen, und deshalb die Nachweisempfindlichkeit des Verfahrens steigern können.

Unter Erfassen wird im Sinne der Offenbarung sowohl der qualitative als auch quantitative Nachweis von makromolekularen Biopolymeren in einem (zu untersuchenden) Analyten verstanden. Dies bedeutet, dass der Begriff "Erfassen" ebenfalls einschließt, die Abwesenheit von makromolekularen Biopolymeren im Analyten festzustellen.

Unter "Einheit zur Immobilisierung" wird im Sinne der Offenbarung eine Anordnung verstanden, die eine Oberfläche aufweist, auf der die Fängermoleküle immobilisiert werden können, d.h. an die die Fängermoleküle durch physikalische oder chemische Wechselwirkungen binden können. Diese Wechselwirkungen schließen hydrophobe oder ionische (elektrostatische) Wechselwirkungen und kovalente Bindungen ein. Beispiele für geeignete Oberflächen-Materialien, die für die mindestens eine Einheit zur Immobilisierung verwendet werden können, sind Metalle wie Gold oder Silber, Kunststoffe wie Polyethylen- oder Polypropylen oder anorganische Stoffe wie Siliziumdioxid, z.B. in Form von Glas.

Ein Beispiel für eine physikalische Wechselwirkung, die eine Immobilisierung der Fängermoleküle bewirkt, ist eine Adsorption an der Oberfläche. Diese Art der Immobilisierung kann beispielsweise stattfinden, wenn das Mittel zur Immobilisierung ein Kunststoffmaterial ist, das für die Herstellung von Mikrotiterplatten verwendet wird (z.B. Polypropylen). Allerdings ist eine kovalente Verknüpfung der Fängermoleküle an die Einheit zum Immobilisieren bevorzugt, weil dadurch die Orientierung der Fängermoleküle gesteuert werden kann. Die kovalente Verknüpfung kann über jede geeignete Verknüpfungschemie ("Linker-Chemie") erfolgen. In einer Ausgestaltung des Verfahrens ist die mindestens eine Einheit zum Immobilisieren auf einer Elektrode oder einer Photodiode aufgebracht.

Die Erfassungseinheit des hier verwendeten Biosensor ist als in dem Substrat integrierte Schaltung ausgestaltet. Dies bedeutet, dass die Einheit zum Immobilisieren entweder auf demselben Substrat angeordnet ist oder in diesem (gemeinsamen) Substrat integriert ist. Dies schließt die Möglichkeit ein, dass die mindestens Einheit zum Immobilisieren z.B. in einer Aufnahmeeinheit für das Substrat angeordnet ist. Ein Beispiel für ein geeignetes Substrat ist z.B. ein Halbleiter-Chip, insbesondere ein CMOS-Chip oder Silicium-Wafer. Eine Aufnahmeeinheit kann z.B. ein Gehäuse sein oder eine Halterung, die z.B. ein Substrat wie einen Halbleiterchip aufnimmt. Dabei kann die Erfassungseinheit in jeder beliebigen räumlichen Orientierung zu der Einheit zum Immobilisieren angeordnet sein, die es ermöglicht, ein Chemilumineszenzsignal zu erfassen, das auf/oberhalb bzw. in der unmittelbaren Umgebung der Einheit zum Immobilisieren von der Markierung erzeugt wird. Als Substrat ist generell jedes Material, insbesondere jedes Halbleitermaterial, geeignet, in das die Erfassungseinheit als Schaltung eingebettet werden kann.

In dem Verfahren wird das Chemilumineszenzsignal von einer Markierung erzeugt, die sich an den Fängermolekülen befindet. Dies bietet insbesondere den Vorteil, dass zu erfassende makromolekulare Biopolymere nicht zuvor für ihren Nachweis markiert werden müssen. Dadurch wird die Gefahr vermieden, dass möglicherweise ein Teil der Probe oder eventuell die gesamte Probe bei der Markierungsreaktion verloren geht, oder dass die Markierungsreaktion nicht vollständig abläuft und so das Ergebnis verfälscht.

Bei dieser Ausgestaltung werden Fängermoleküle, an die keine zu erfassenden makromolekularen Biopolymere gebunden haben, entfernt, bevor die Markierung zur Erzeugung des Chemilumineszenzsignals angeregt wird.

Als makromolekulare Biopolymere können mit dem vorliegenden Verfahren insbesondere Nukleinsäuren, Oligonukleotide, Proteine oder Komplexe aus Nukleinsäuren und Proteinen erfasst werden.

Bei einer bevorzugten Ausführungsform des Verfahrens ist die mindestens eine Einheit zum Immobilisieren auf der Erfassungseinheit, z.B. direkt oberhalb der Erfassungseinheit, aufgebracht.

Bei einer Weiterbildung sind mehrere Einheiten zum Immobilisieren in einer regelmäßigen Anordnung auf mehreren Erfassungseinheiten aufgebracht.

Bevorzugt ist ein Verfahren, bei dem als die Erfassungseinheit oder die mehreren Erfassungseinheiten eine Photodiode, eine CCD-Kamera oder eine CMOS-Kamera verwendet wird. In einer bevorzugten Ausführungsform wird als Erfassungseinheit eine Photodiode eingesetzt.

Bei einer Ausgestaltung des Verfahrens wird zur Erfassung der Biopolymere ein elektrisches Signal verwendet, das die Folge der ersten Stufe der Erfassungseinheit ist, die das Chemilumineszenzsignal in ein elektrisches Signal umsetzt. Allgemein gesagt wird dieses elektrische Signal also indirekt durch das Chemilumineszenzsignal hervorgerufen.

Dieses elektrische Signal (in) der Erfassungseinheit ist vorzugsweise ein elektrischer Strom wie ein Photostrom oder eine Spannung wie eine Photospannung bei der Photodiode. Die Erfassung des optischen Chemilumineszenzsignals, z.B. durch Integration des elektrischen Signals, wird bevorzugt über mehrere Minuten durchgeführt.

Insbesondere im Falle von Mehrfach- oder ParallelBestimmungen ist es bei dem Verfahren vorteilhaft, das jede einzelne Erfassungseinheit zur Erfassung des elektrischen Signals individuell ansteuerbar ist. Dadurch wird eine Verfälschung des Messergebnis z.B. durch einfallende Streustrahlung benachbarter Sensorfelder vermieden.

An dieser Stelle sei darauf hingewiesen, dass die hier offenbarte Gestaltung des Biosensors wegen des Wegfalls einer außerhalb des Reaktionsbereichs angeordneten Erfassungsvorrichtung wie einem Konfokal-Mikroskop oder einem Röntgenfilm nicht nur einen vereinfachten Aufbau bietet. Vielmehr ermöglicht der hier beschriebene Aufbau eine kontinuierliche Messung für jede Einheit zum Immobilisieren. Dies ist insbesondere wichtig und vorteilhaft, wenn z.B. Vorgänge, die die Reaktionsdynamik oder -kinetik betreffen, untersucht werden sollen.

In einer weiteren vorteilhaften Ausgestaltung des Verfahrens wird als Einheit zum Immobilisieren von makromolekularen Biopolymeren ein Nanopartikel verwendet.

Unter einem Nanopartikel wird im Sinne der Offenbarung ein Partikel verstanden, der durch sogenannte Nanostrukturierungs-Verfahren erhalten werden kann. Nanostruktiarierungs-Verfahren, die zur Erzeugung solcher Nanopartikel auf geeigneten Substraten herangezogen werden können, sind beispielsweise die in [7] und [8] beschriebene Verwendung von Block-Copolymer-Microemulsionen oder die in [9] beschriebenen Verwendung von Kolloidpartikeln als Strukturierungsmasken. Das in [9] beschriebene Verfahren ist im Prinzip analog zu einem im Bereich der Substrat-Strukturierung üblicherweise eingesetzten Lithographie-Verfahren. Daher sei hier betont, das ein Nanopartikel im Sinne der Offenbarung folglich nicht auf diejenigen Partikel beschränkt ist, die durch eines der hier beispielhaft genannten Verfahren erhalten werden. Vielmehr ist ein solcher Nanopartikel jeder Partikel, dessen Durchmesser im Nanometer-Bereich liegt, d.h. allgemein im Bereich von 2 bis 50 nm, vorzugsweise im Bereich von 5 bis 20 nm, besonders bevorzugt im Bereich von 5 bis 10 nm.

Eine "Einheit zur Immobilisierung, die ein Nanopartikel ist", die im folgenden auch nanopartikelförmige Einheit genannt wird, ist folglich ein oben beschriebener Nanopartikel, der eine Oberfläche aufweist, auf der die Fängermoleküle immobilisiert werden können, d.h. die Oberfläche ist so beschaffen, dass an ihr die Fängermoleküle durch physikalische oder chemische Wechselwirkungen binden können. Diese Wechselwirkungen schließen hydrophobe oder ionische (elektrostatische) Wechselwirkungen und kovalente Bindungen ein. Beispiele für geeignete Oberflächen-Materialien, die für die mindestens eine nanopartikelförmige Einheit zur Immobilisierung verwendet werden können, sind Metalle wie Gold oder Silber, halbleitende Materialien wie Silizium, Kunststoffe wie Polyethylen- oder Polypropylen oder Siliziumdioxid, z.B. in Form von Glas. Nanopartikelförmige Einheiten aus Kunststoffen und Siliziumdioxid sind dabei durch Verwendung der in [9] beschriebenen Kolloidmasken-Verfahren erhältlich. Nanopartikelförmige Einheiten aus halbleitenden Materialien wie Silizium können beispielsweise auch durch das Stranski-Krastanov-Verfahren gebildet werden. Durch Oxidation solcher Nanopartikel aus Silizium sind weiterhin nanopartikelförmige Einheiten aus Siliziumdioxid erhältlich.

Aufgrund der oben beschriebenen Herstellungsverfahren nehmen nanopartikelförmige Einheiten zum Immobilisieren, die auf geeigneten Substratoberflächen (Halte-Bereiche), beispielweise von Photodioden oder CCDs, aufgebracht sind, eine regelmäßige Anordnung ein, mit Abständen voneinander im Bereich einiger 10 Nanometer, beispielsweise von ca. 10 bis 30 nm auf diesen Oberflächen. Die Art der Anordnung und der Abstand der Nanopartikel voneinander hängt ebenso wie die Größe der Nanopartikel von dem jeweiligen Verfahren zur Ausbildung der Nanopartikel ab.

Ein Vorteil bei der Verwendung von nanopartikelförmigen Einheiten zum Immobilisieren besteht darin, dass auf diesen Nanopartikeln eine genau definierte Anzahl von Fängermolekülen immobilisiert werden kann. Dies ist insbesondere bei der quantitativen Erfassung von makromolekularen Biopolymeren mittels des vorliegenden Verfahrens von Vorteil. Ein weiterer Vorteil bei der Verwendung von Nanopartikeln als Einheiten zum Immobilisieren bietet sich dadurch, dass durch den Abstand der Nanopartikel untereinander, d.h. die räumliche Trennung der Fängermoleküle, eine bessere räumliche Zugänglichkeit der Fängermoleküle für die daran bindenden makromolekularen Biopolymere gegeben ist und somit die Wahrscheinlichkeit einer Wechselwirkung erhöht wird. Die Ausbildung als Nanopartikel vergrößert zudem die effektive Oberfläche.

Unter makromolekularen Biopolymeren werden hier beispielsweise (längerkettige) Nukleinsäuren wie DNA-Moleküle, RNA-Moleküle, PNA-Moleküle oder cDNA-Moleküle oder kürzere Oligonukleotide mit z.B. 10 bis 50 Basenpaaren (bp), insbesondere 10 bis 30 bp verstanden. Die Nukleinsäuren können doppelsträngig sein, jedoch auch zumindest einzelsträngige Bereiche aufweisen oder, zum Beispiel durch vorangehende thermische Denaturierung (Strangtrennung) für ihren Nachweis, als Einzelstränge vorliegen. Die Sequenz der zu erfassenden Nukleinsäuren kann dabei zumindest teilweise oder vollständig vorgegeben, d.h. bekannt sein. Weitere makromolekulare Biopolymere sind Proteine oder Peptide. Diese können aus den üblicherweise in Proteinen vorkommenden 20 Aminosäuren aufgebaut sein, aber auch natürlich nicht vorkommende Aminosäuren enthalten oder z.B. durch Zuckerreste (Oligosaccharide) modifiziert sein oder post-translationale Modifikationen enthalten. Ferner können auch Komplexe aus mehreren unterschiedlichen makromolekularen Biopolymeren erfasst werden, beispielsweise Komplexe aus Nukleinsäuren und Proteinen.

Sollen als makromolekulare Biopolymere Proteine oder Peptide erfasst werden, so werden als Fängermoleküle bevorzugt Liganden verwendet, die die zu erfassenden Proteine oder Peptide spezifisch binden können. Die Fängermoleküle/Liganden sind vorzugsweise durch kovalente Bindungen mit der Einheit zur Immobilisierung verknüpft.

Als Liganden für Proteine und Peptide kommen niedermolekulare Enzymagonisten oder Enzymantagonisten, Pharmazeutika, Zucker oder Antikörper oder andere geeignete Moleküle in Betracht, die die Fähigkeit besitzen, Proteine oder Peptide spezifisch zu binden.

Wenn DNA-Moleküle (Nukleinsäuren oder Oligonukleotide) einer vorgegeben Nukleotidsequenz mit dem hier beschriebenen Verfahren erfasst werden, so werden sie vorzugsweise in einzelsträngiger Form erfasst, d.h. sie werden ggf. vor der Erfassung durch Denaturierung wie vorstehend erläutert in Einzelstränge überführt. In diesem Fall werden als Fängermoleküle dann vorzugsweise DNA-Sondenmoleküle mit einer zu dem einzelsträngigen Bereich komplementären Sequenz verwendet. Die DNA-Sondenmoleküle können wiederum Oligonukleotide oder auch längere Nukleotidsequenzen aufweisen, solange diese keine der intermolekularen Strukturen ausbilden, die eine Hybridisierung des Sondenmoleküls mit der zu erfassenden Nukleinsäure verhindern. Allerdings ist es auch möglich, DNA-bindende Proteine oder Agenzien als Fängermolekül einzusetzen.

Anzumerken ist, dass es selbstverständlich möglich ist, mit dem vorliegenden Verfahren nicht nur eine einzige Art von Biopolymeren in einer einzelnen Messreihe zu erfassen. Vielmehr können mehrere makromolekulare Biopolymere gleichzeitig oder auch nacheinander erfasst werden. Dazu können auf der Einheit zum Immobilisieren mehrere Arten von Fängermolekülen, von denen jedes eine (spezifische) Bindungsaffinität für ein bestimmtes zu erfassendes Biopolymer aufweist, gebunden werden, und/oder es können mehrere Einheiten zum Immobilisieren eingesetzt werden, wobei an jeder von diesen Einheiten nur eine Art von Fängermolekül gebunden wird. Bei diesen Mehrfachbestimmungen wird für jedes zu erfassende makromolekulare Biopolymer vorzugsweise eine von den anderen Markierungen durch die Wellenlänge des ausgesandten Chemilumineszenzsignals unterscheidbare Markierung verwendet.

In einem ersten Verfahrensschritt wird die zumindest eine Einheit zum Immobilisieren mit den Fängermolekülen versehen, wobei diese in einer Ausgestaltung des Verfahrens eine Markierung aufweisen, mittels der das detektierbare Signal erzeugt werden kann.

Eine zu untersuchende Probe, vorzugsweise ein flüssiges Medium wie ein Elektrolyt, wird dann mit der Einheit zum Immobilisieren in Kontakt gebracht. Dies erfolgt in der Weise, dass die makromolekularen Biopolymere an die Fängermoleküle binden können. Für den Fall, dass sich in dem Medium mehrere zu erfassende makromolekulare Biopolymere befinden, werden die Bedingungen so gewählt, dass diese jeweils zur gleichen Zeit oder nacheinander an ihre entsprechendes Fängermolekül binden können.

Nachdem eine ausreichende Zeitdauer gewartet worden ist, damit die makromolekularen Biopolymere an das entsprechende Fängermolekül bzw. die entsprechenden Fängermoleküle binden konnten, werden nicht gebundene Fängermoleküle von der Einheit bzw. den Einheiten zum Immobilisieren, auf der bzw. auf denen sie sich befinden, entfernt. Weil die Fängermoleküle mit der Markierung versehen sind, ist dieser Schritt obligatorisch.

Sollen als makromolekulare Biopolymere Proteine oder Peptide erfasst werden, so werden die nicht gebundenen, vorzugsweise über eine kovalente Bindung immobilisierten und als Fängermoleküle verwendeten Liganden von der mindestens einen Einheit zum Immobilisieren entfernt, indem ein Material mit der mindestens einen Einheit zum Immobilisieren in Kontakt gebracht wird. Dabei ist das Material imstande, die chemische Verbindung zwischen dem Liganden und der Einheit zum Immobilisieren zu hydrolysieren.

Für den Fall, dass die Fängermoleküle niedermolekulare Liganden sind, lassen sich diese, falls ungebunden, auch enzymatisch entfernen.

Hierzu sind die Liganden über eine enzymatisch spaltbare Verbindung kovalent mit der Einheit zur Immobilisierung verbunden, beispielsweise über eine Esterverbindung.

In diesem Falle kann beispielsweise eine Carboxylester-Hydrolase (Esterase) eingesetzt werden, um ungebundene Ligandenmoleküle zu entfernen. Dieses Enzym hydrolysiert diejenige Esterbindung zwischen der Einheit zur Immobilisierung und dem jeweiligen Ligandenmolekül, das nicht von einem Peptid oder Protein gebunden wurde. Dagegen bleiben die Esterverbindungen zwischen der Einheit zum Immobilisieren und denjenigen Molekülen, die eine Bindungswechselwirkung mit Peptiden oder Proteinen eingegangen sind, aufgrund der verminderten sterischen Zugänglichkeit, die durch die Raumerfüllung des gebundenen Peptids oder Proteins eintritt, unversehrt.

Für den Fall, dass die Fängermoleküle DNA-Stränge sind, erfolgt die Entfernung der nicht gebundenen Fängermoleküle enzymatisch, beispielsweise mit Hilfe eines Enzyms mit Nukleaseaktivität. Vorzugsweise wird als Enzym mit Nukleaseaktivität ein Enzym verwendet, das selektiv einzelsträngige DNA abbaut. Hierbei ist die Selektivität des abbauenden Enzyms für einzelsträngige DNA zu berücksichtigen. Besitzt das für den Abbau nicht hybridisierter DNA-Einzelstränge ausgewählte Enzym diese Selektivität nicht, so wird möglicherweise auch die zu erfassende DNA, die in Form eines doppelsträngigen Hybrids mit dem Fängermolekül vorliegt, unerwünschterweise ebenfalls abgebaut.

Insbesondere können zum Entfernen der nicht gebundenen DNA-Fängermoleküle, die nachfolgend auch als Sondenmoleküle bezeichnet werden, von der jeweiligen Elektrode DNA Nukleasen, beispielsweise eine Nuklease aus Mung-Bohnen, die Nuklease P1 oder die Nuklease S1 verwendet werden. Ebenfalls können DNA-Polymerasen, die aufgrund ihrer
5'→ 3' Exonukleaseaktivität oder ihrer
3'→ 5' Exonukleaseaktivität imstande sind, einzelsträngige DNA abzubauen, verwendet werden.

Nach dem Entfernen der nicht gebundenen Fängermoleküle werden die makromolekularen Biopolymere mittels der Markierung von der Erfassungseinheit erfasst. Der hier für diesen Zweck verwendete Biosensor ist derart gestaltet, dass die Messung ortsaufgelöst direkt auf dem Sensor erfolgt, indem z.B. die Einheit zur Immobilisierung direkt auf einer zur Messung verwendeten Photozelle aufgebracht ist. Weiterhin können in dem Sensor Schaltungselemente für das individuelle Ansteuern jeder Erfassungseinheit sowie eine entsprechende Auswerte- und Signalverarbeitungseinheit aufweisen. Dabei kann diese Auswerteinheit neben dem Integrator für das elektrische Signal z.B. einen Analog/Digitalwandler und/oder einen Vorverstärker für die Erfassung des elektrischen Signals aufweisen. Vorzugsweise sind alle diese Einheiten in den Sensor integriert.

Dies hat den Vorteil einer vereinfachten Messanordnung. Eine solche Messanordnung kann z.B. mittels einer CMOS-Kamera oder einem CCD realisiert werden.

Bei dem Verfahren kann als Referenzmessung eine Messung des Signals durchgeführt werden, bevor oder nachdem die mindestens eine Einheit zum Immobilisieren von makromolekularen Biopolymeren mit den Fängermolekülen versehen wird. Anschließend wird die zur Erfassung dienende Messung durchgeführt. In beiden Fällen werden die ermittelten Werte aus den beiden Messung des erhaltenen (elektrischen) Signals miteinander verglichen. Wenn die Signalintensität der gemessenen Werte sich in einer Weise unterscheiden, dass die Differenz der ermittelten Werte größer ist als ein vorgegebener Schwellenwert, so wird angenommen, dass makromolekulare Biopolymere an Fängermoleküle gebunden haben und dadurch die Veränderung der Intensität des am Empfänger empfangenen Signals verursacht worden ist. Ist der ermittelte Wert kleiner als der Schwellenwert, wird andererseits angenommen, dass keine Biopolymere an die Fängermoleküle gebunden haben, d.h. auch nicht in einer untersuchten Probe vorhanden waren.

Ausführungsbeispiele der Offenbarung sind in den Figuren dargestellt und werden im weiteren näher erläutert.

Es zeigen
- Figuren 1a bis 1c: einen Biosensor zu unterschiedlichen Verfahrenszuständen, anhand denen das Verfahren gemäß einem Ausführungsbeispiel der Offenbarung erläutert wird;
- Figuren 2a bis 2c: einen Biosensor zu unterschiedlichen Verfahrenszuständen, anhand denen das Verfahren gemäß einem weiteren Ausführungsbeispiel der Offenbarung erläutert wird;
- Figuren 3a bis 3f: einen Biosensor, mit dem eine weitere Ausführungsform der hier beschriebenen Verfahren durchgeführt werden kann;
- Figuren 4a und 4b: eine Skizze zweier Planarelektroden, mittels derer die Existenz zu erfassender DNA-Stränge in einem Elektrolyt (Figur 2a) bzw. deren Nichtexistenz (Figur 2b) nachgewiesen werden können

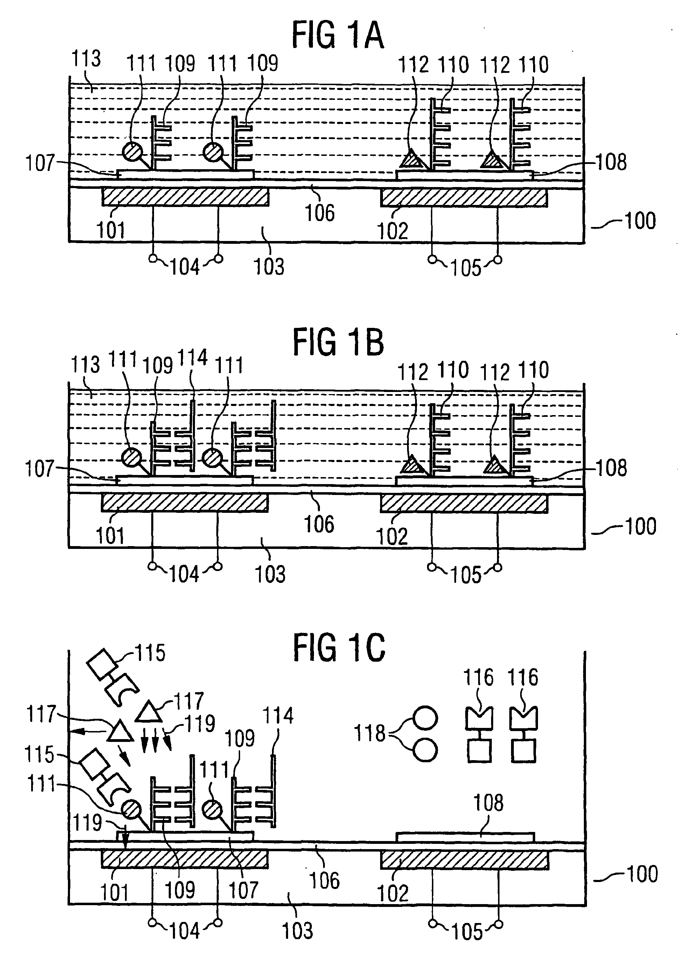

**Fig.1** zeigt einen Ausschnitt aus einem Biosensors 100, mit dem ein erstes Ausführungsbeispiel des hier beschriebenen Verfahren durchgeführt werden kann.
**Fig.1a** zeigt den Biosensor 100 mit einer ersten Photodiode 101 und einer zweiten Photodiode 102, die in einer Schicht 103 aus Halbleitermaterial angeordnet sind.

Die erste Photodiode 101 und die zweite Photodiode 102 sind über erste elektrischen Anschlüsse 104 bzw. zweite elektrische Anschlüsse 105 mit einer Auswerteeinheit (nicht dargestellt) verbunden. Diese Auswerteeinheit kann in dem Sensor 100 integriert sein. Die beiden Photodioden 101, 102 sind ferner mit einer Oxidschicht 106 und einer ersten Einheit 107 zum Immobilisieren von makromolekularen Biopolymeren bzw. einer zweiten Einheit 108 zum Immobilisieren von makromolekularen Biopolymeren versehen. Die Einheiten zum Immobilisieren 107 und 108 sind aus Gold hergestellt.

Alternativ können die Einheiten 107, 108 zum Immobilisieren auch aus Siliziumoxid hergestellt sein und mit einem Material beschichtet werden, das geeignet ist, Fängermoleküle zu immobilisieren.

Beispielsweise können bekannte Alkoxysilanderivate verwendet werden wie
- 3-Glycidoxypropylmethyloxysilan,
- 3-Acetoxypropyltrimethoxysilan,
- 3-Aminopropyltriethoxysilan,
- 4-(Hydroxybutyramido)propyltriethoxysilan,
- 3-N,N-bis(2-hydroxyethyl)aminopropyltriethoxysilan,
oder andere artverwandte Materialien, die imstande sind, mit ihrem einen Ende eine Bindung, z.B. eine kovalente Bindung, mit der Oberfläche des Siliziumoxids einzugehen und mit ihrem anderen Ende dem zu immobilisierenden Sondenmolekül eine chemisch reaktive Gruppe wie einen Epoxy-, Acetoxy-, Amin- oder Hydroxylrest zur Reaktion anzubieten. Alternativ kann z.B. Poly-L-Lysin verwendet werden.

Reagiert ein zu immobilisierendes Fängermolekül mit einer solchen aktivierten Gruppe, so wird es über das gewählte Material als eine Art kovalenter Linker auf der Oberfläche der Beschichtung auf der Einheit zum Immobilisieren gebunden.

Auf den Einheiten zum Immobilisieren 107 und 108 werden DNA-Sondenmoleküle 109, 110 als Fängermoleküle aufgebracht.

Dabei sind auf der ersten Photodiode 101 mittels der Einheit 107 erste DNA-Sondenmoleküle 109 mit einer zu einer vorgegebenen ersten DNA-Sequenz komplementären Sequenz aufgebracht. Die DNA-Sondenmoleküle 109 sind jeweils mit einer ersten ein Chemilumineszenzsignal erzeugenden Markierung 111 versehen.

Als Markierung kann Biotin verwendet werden, das sowohl am 5'- als auch am 3'-Terminus eines DNA-Fängermoleküls 109 eingebaut werden kann; vgl. [10).

Auf der zweiten Photodiode 102 sind zweite DNA-Sondenmoleküle 110 mit einer Sequenz, die komplementär ist zu einer vorgegebenen zweiten DNA-Sequenz ist, aufgebracht. Die DNA-Sondenmoleküle 110 sind jeweils mit einer zweiten Markierung 112 markiert. Als Markierung 112 kann dabei z.B. Digoxigenin eingesetzt werden; vgl. [11].

An die Purinbasen Adenin (A), Guanin (G), und Pyrimidinbasen Thymin (T) bzw. Uracil (U) im Falle einer oben beschriebenen Markierung, oder Cytosin (C), können jeweils zu den Sequenzen der Sondenmoleküle komplementäre Sequenzen von DNA-Strängen in der üblichen Weise, d.h. durch Basenpaarung über Wasserstoffbrückenbindungen zwischen A und T oder bzw. zwischen C und G, hybridisieren. Bei Verwendung anderer Nukleinsäuremoleküle werden entsprechend andere Basen, im Falle eines RNA-Moleküls beispielsweise Uridin (U) verwendet.

**Fig.1a** zeigt ferner einen Elektrolyten 113, der mit den Photodioden 101, 102 und den DNA-Sondenmolekülen 108, 109 in Kontakt gebracht wird.

**Fig.1b** zeigt den Biosensor 100 für den Fall, dass in dem Elektrolyten 113 DNA-Stränge 114 enthalten sind, die eine vorgegebene erste Nukleotidsequenz aufweisen, die komplementär ist zu der Sequenz der ersten DNA-Sondenmoleküle 109.

In diesem Fall hybridisieren die zu den ersten DNA-Sondenmolekülen 109 komplementären DNA-Stränge 114 mit den ersten DNA-Sondenmolekülen 109, die auf der ersten Photodiode 101 aufgebracht sind.

Da die Sequenzen von DNA-Strängen nur mit der jeweils spezifischen Komplementärsequenz hybridisieren, hybridisieren die zu den ersten DNA-Sondenmolekülen komplementären DNA-Stränge nicht mit den zweiten DNA-Sondenmolekülen 110.

Wie aus **Fig.1b** ersichtlich ist, ist das Resultat nach erfolgter Hybridisierung, dass sich auf der ersten Photodiode 101 hybridisierte Moleküle befinden, d.h. doppelsträngige DNA-Moleküle immobilisiert sind. Auf der zweiten Photodiode 102 sind nur die zweiten DNA-Sondenmoleküle 110 als weiterhin einzelsträngige Moleküle vorhanden.

In einem weiteren Schritt wird mittels eines (biochemischen) Verfahrens, beispielsweise durch Zugabe von DNA-Nukleasen zu dem Elektrolyten 113, eine Hydrolyse der einzelsträngigen DNA-Sondenmoleküle 110 auf der zweiten Photodiode 102 bewirkt.

Hierbei ist die Selektivität des abbauenden Enzyms für einzelsträngige DNA zu berücksichtigen. Besitzt das für den Abbau der nicht hybridisierten DNA-Einzelstränge ausgewählte Enzym diese Selektivität nicht, so wird möglicherweise die als doppelsträngige DNA vorliegende zu erfassende Nukleinsäure ebenfalls (unerwünschterweise) abgebaut, was zu einer Verfälschung des Messergebnisses führen würde.

Nach Entfernen der einzelsträngigen DNA-Sondenmoleküle, d.h. der zweiten DNA-Sondenmoleküle 110 auf der zweiten Photodiode 102 sind lediglich die Hybride aus den zu erfassenden DNA-Molekülen 114 und den dazu komplementären ersten DNA-Sondenmolekülen 109 (vgl. **Fig.1c****)** vorhanden.

Beispielsweise kann zum Entfernen der nicht gebundenen einzelsträngigen DNA-Sondenmoleküle 110 auf der zweiten Photodiode 102, d.h. der zweiten Einheit zum Immobilisieren, einer der folgenden Stoffe zugegeben werden:
- Nuklease aus Mung-Bohnen,
- Nuklease P1, oder
- Nuklease S1.

Zu diesem Zweck können ebenfalls DNA-Polymerasen, die aufgrund ihrer 5'→ 3' Exonukleaseaktivität oder ihrer 3'→ 5' Exonukleaseaktivität imstande sind, einzelsträngige DNA abzubauen, verwendet werden.

Danach wird ein für die Markierung 111 bzw. 112 geeignetes chemisches Nachweiskonjugat 115, 116 zusammen mit dem entsprechenden jeweiligen Substrat 117, 118 und ggf. Signalverstärkern hinzugegeben, um so ein Chemilumineszenz-Signal zu erzeugen **(****Fig.1c****;** wobei dort der Elektrolyt 113 bzw. das für die Nachweisreaktion erforderliche Reaktionsmedium der Übersichtlichkeit halber nicht gezeigt ist). Im Falle der Biotin-Markierung 111 kann z.B. ein Konjugat 115 aus Streptavidin mit Meerrettich-Peroxidase, und als Substrat Luminol und 4-Iodphenol als Signalverstärker-Reagenz verwendet werden. Für die Digoxigenin-Markierung 112 kann z.B. ein sogenanntes "Anti-Digoxingenin-Alkalische-Phosphatase-Konjugat" mit CSPD^{®} als Chemilumineszenz-Substrat eingesetzt werden [11]. Je nach Kompatibilität der Reaktionsbedingungen können die Nachweisreaktionen parallel oder nacheinander ablaufen. Für die Durchführung paralleler Bestimmungen kann der Biosensors auch so gestaltet werden, dass z.B. durch Stege und Wände eine räumliche Abtrennung in separate Reaktionskammern erreicht wird.

Nur im Falle der Markierung 111 wird durch die Zugabe des Nachweiskonjugats das an den ersten DNA-Sondenmolekülen 109 befindliche Markierung zur Erzeugung des ChemilumineszenzSignals angeregt, da die ungebundenen zweiten DNA-Sondenmoleküle 110 samt der Markierung 112 von der zweiten Photodiode 102 durch die Nukleasebehandlung entfernt worden sind (vgl. **Fig.1c****).** Die durch den Pfeil 119 symbolisierte emittierte Chemilumineszenzstrahlung wird von der ersten Photodiode 101 erfasst. An der zweiten Photodiode 102 wird hingegen keine Chemilumineszenzstrahlung detektiert.

Auf diese Weise wird die Anwesenheit der DNA-Moleküle 114 ermittelt. Die Verwendung des hier beschriebenen Biosensors 100 erlaubt eine örtlich aufgelöste Erfassung und bietet eine deutliche Vereinfachung der gesamten Messanordnung, da keine externe Einheit zur Erfassung eines optischen Signals wie Fluoreszenz- oder Chemilumineszenzstrahlung notwendig ist.

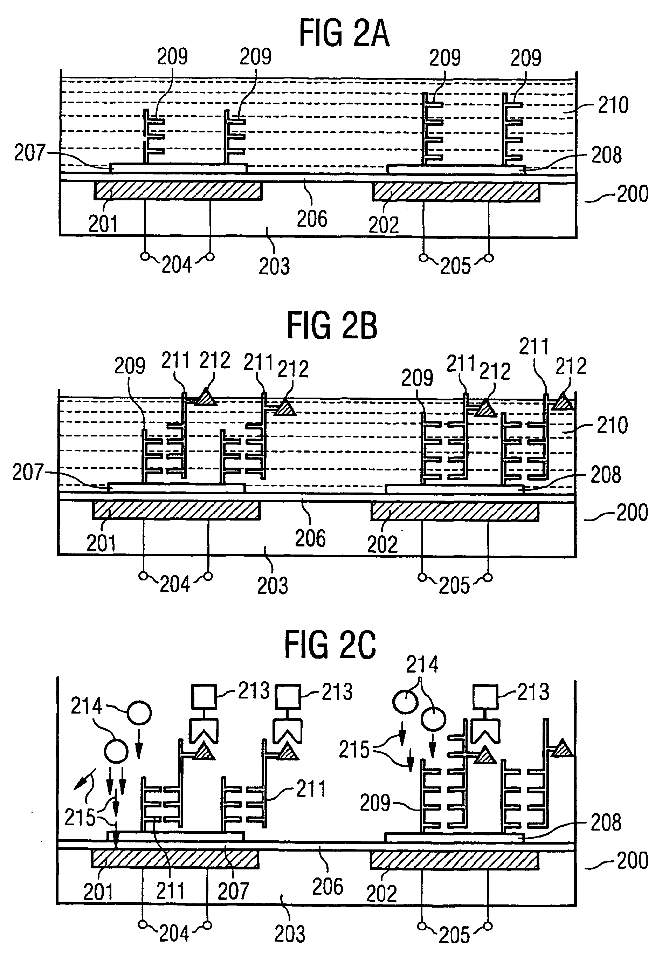

**Fig.2a** zeigt den Biosensor 200, dessen Aufbau identisch mit dem des Biosensors 100 ist. Dies heißt, der Sensor 200 weist eine erste Photodiode 201 und einer zweiten Photodiode 202 auf, die in einer Schicht 203 aus Halbleitermaterial angeordnet sind.

Die erste Photodiode 201 und die zweite Photodiode 202 sind über erste elektrischen Anschlüsse 204 bzw. zweite elektrische Anschlüsse 205 mit einer Auswerteeinheit (nicht dargestellt) verbunden. Diese Auswerteeinheit kann in dem Sensor 200 integriert sein. Die beiden Photodioden 201, 202 sind ferner mit einer Oxidschicht 206 und einer ersten Einheit 207 zum Immobilisieren von makromolekularen Biopolymeren bzw. einer zweiten Einheit 208 zum Immobilisieren von makromolekularen Biopolymeren versehen. Die Einheiten zum Immobilisieren 207 und 208 sind z.B. aus Gold oder Silizium hergestellt. Auf ihnen werden DNA-Sondenmoleküle 209 auf die oben beschriebene Weise als Fängermoleküle aufgebracht.

Dabei sind in diesem Beispiel sowohl auf der ersten Photodiode 201 als auf der zweiten Photodiode mittels der Einheit 207 bzw. 208 erste DNA-Sondenmoleküle 209 mit einer zu einer vorgegebenen (ersten) DNA-Sequenz komplementären Sequenz aufgebracht.

**Fig.2a** zeigt ferner einen Elektrolyten 210, der mit den Photodioden 201, 202 und den DNA-Sondenmolekülen 209 in Kontakt gebracht wird.

**Fig.2b** zeigt den Biosensor 200 für den Fall, dass in dem Elektrolyten 210 DNA-Moleküle 211 enthalten sind, die eine vorgegebene erste Nukleotidsequenz aufweisen, die komplementär zu der Sequenz der ersten DNA-Sondenmoleküle 209 ist. Die DNA-Moleküle 211 tragen als Markierungen 212 z.B. die oben beschriebene Digoxigenin- oder Biotin-Markierung.

In diesem Fall bilden sich Hybride aus den DNA-Sondenmolekülen 209 und den zu ihnen komplementären DNA-Molekülen 211 auf den Photodiode 201, 202 auf **(****Fig.2b****).**

Nach einem ggf. vorgenommenen Waschschritt wird ein für die Markierung 212 geeignetes Nachweis-Konjugat 213 nebst Substrat 214 und ggf. Signalverstärkungsverbindung wie oben beschrieben hinzugegeben, um ein Chemilumineszenz-Signal zu erzeugen.

Dabei kommt es zu der durch den Pfeil 215 symbolisierten Emission von Chemilumineszenzstrahlung und deren Erfassung durch die Photodioden 201, 202. Auf diese Weise wird die Anwesenheit der DNA-Moleküle 211 ermittelt.

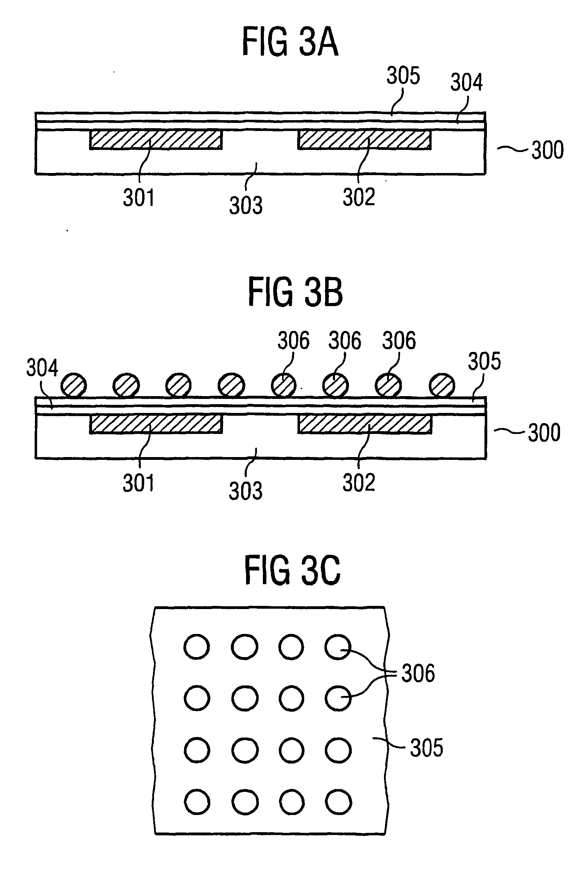

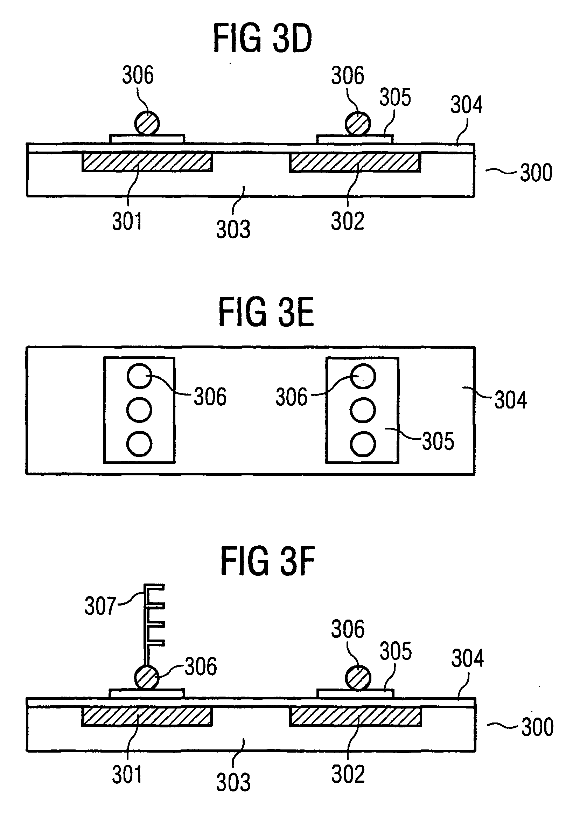

**Fig.3** zeigt einen Ausschnitt eines Biosensors 300, der mit mindestens einer Einheit zur Immobilisierung in Form von Nanopartikeln ausgestaltet ist, und mit dem eine weitere Ausführungsform des hier beschriebenen Verfahrens durchgeführt werden kann.

Der Biosensor 300 weist eine erste Photodiode 301 und eine zweite Photodiode 302 auf, die in einer Schicht 303 aus Halbleitermaterial wie Silizium angeordnet sind. Der Biosensor 300 weist weiterhin eine Oxidschicht 304 und eine zweite darauf befindliche Schicht 305 auf. Die zweite Schicht 305 besteht dabei aus einem Metall, das nicht für die Immobilisierung von makromolekularen Biopolymeren geeignet ist. Die Schicht 305 kann z.B. aus Platin gebildet werden.

Auf der Schicht 305 werden die Einheiten zum Immobilisieren von makromolekularen Biopolymeren, die die Form von Nanopartikeln aufweisen, durch folgendes Verfahren gebildet.

Eine Lösung von 0,5 Gew.-% Block-Copolymer Polystryol(PS)-block-poly(2-vinylpyridin)(P2VP) der allgemeinen Formel PS(x)-b-P2VP(y) wird, wie in [7] und [8] beschrieben, mit 0,5

Äquivalenten HAuCl₄·H₂O pro Pyridin-Einheit zur Ausbildung von monodispersen (micellar gelösten) Gold-Partikeln versetzt. x und y geben in der Formel die Anzahl der Grundeinheiten entsprechend dem Verhältnis zwischen Monomer und Initator an.

Nach der Bildung homogener Micellen wird aus dieser Lösung durch Reduktion mit Hydrazin, wie in [7] und [8] beschrieben, eine Monoschicht von Nanopartikeln aus Gold auf der Schicht 305 abgeschieden. Anschließend werden die organischen Bestandteile der abgeschiedenen Micellen, d.h. das Block-Copolymer, durch Plasmaätzen mittels eines Sauerstoffplasmas von der Schicht 305 entfernt (vgl. [8]). Die Goldpartikel 306, die als die Einheiten zum Immobilisieren von makromolekularen Biopolymeren dienen, bleiben bei dieser Behandlung mit Plasma unversehrt und bilden, wie in der Schnittansicht von **Fig.3b** und der Draufsicht von **Fig.3c** veranschaulicht, eine regelmäßige Anordnung auf der Schicht 305 aus (vgl. [7]). Die Abstände zwischen den Gold-Nanopartikeln 306 betragen in der Regel einige 10 nm, z.B. ca. 20 bis 30 nm. Die Nanopartikel besitzen vorzugsweise eine Größe im Bereich von ca. 5 bis 10 nm.

Neben den oben genannten Blockpolymeren sind selbstverständlich auch weitere Blockpolymere zur Ausbildung der Nanopartikel verwendbar.

Alternativ können die Einheiten 306 zum Immobilisieren in Nanopartikel-Form auf dem Biosensor erzeugt werden, wie in [9] beschrieben, indem zunächst eine Maske für die Nanostrukturierung aus Kolloidpartikel auf der Schicht 305 ausgebildet wird und dann z.B. mittels Vakuumdeposition Goldpartikel abgelagert werden.

Nach dem Aufbringen der Nanopartikel 306 aus Gold wird der Sensor 300 derart strukturiert, dass die Schicht 305 aus Platin samt den Einheiten 306 zum Immobilisieren nur auf Bereichen zurückbleibt, die sich auf den Photodioden 301, 302 befinden, wie in der Schnittansicht von **Fig.3d** und der Draufsicht von **Fig.3e** gezeigt ist. Diese Strukturierung ist z.B. mit Hilfe jedes geeigneten gängigen chemischen Ätzverfahrens möglich.

Mit Hilfe des so gestalteten Biosensors 300 können z.B. beide in den Ausführungsbeispielen gemäß Figur beschriebenen Verfahren zum Erfassen von makromolekularen Biopolymeren durchgeführt werden. **Fig.3f** zeigt ein auf einem Gold-Nanopartikel 306 mittels der Gold-Schwefel-Kopplung immobilisiertes DNA-Fängermolekül 307. Diese kann, falls gewünscht, mit einer Markierung, mit deren Hilfe ein Chemilumineszenzsignal erzeugt wird, versehen sein.

Die Verwendung des Biosensors 300 bietet den Vorteil, dass die in Nanopartikel-Form vorliegenden Einheiten 305 zum Immobilisieren es ermöglichen, eine genau definierte Anzahl von Fängermolekülen zu immobilisieren. Der Einsatz des Biosensors 300 ist daher bei einer quantitativen Erfassung von makromolekularen Biopolymeren bevorzugt.

In diesem Dokument sind folgende Veröffentlichungen zitiert:
[1] R. Hintsche et al., Microbiosensors Using Electrodes Made in Si-Technology, Frontiers in Biosensorics, Fundamental Aspects, edited by F. W. Scheller et al., Dirk Hauser Verlag, Basel, S. 267 - 283, 1997
[2] R. Hintsche et al., Microelectrode arrays and application to biosensing devices, Biosensors & Bioelectronics, Vol. 9, S. 697 - 705, 1994
[3] M. Paeschke et al,-Voltammetric Multichannel Measurements Using Silicon Fabricated Microelectrode Arrays, Electroanalysis, Vol. 7, Nr. 1, S. 1 - 8, 1996
[4] P. van Gerwen, Nanoscaled Interdigitated Electrode Arrays for Biochemical Sensors, IEEE, International Conference on Solid-State Sensors and Actuators, Chicago, S.907 - 910, 16. - 19. Juni 1997
[5] N.L. Thompson, B.C. Lagerholm, Total Internal Reflection Fluorescence: Applications in Cellular Biophysics, Current Opinion in Biotechnology, Vol. 8, S. 58 - 64, 1997.
[6] Roche Molecular Biochemicals, 1999 Biochemicals Catalog, S.99.
[7] J.P. Spatz et al., Mineralization of Gold Nanoparticles in a Block Gold Copolymer Microemulsion, Chem. Eur. J., Vol. 2, S. 1552 - 1555, 1996
[8] J.P. Spatz et al., Ordered Deposition of Inorganic Cluster from Micellar Block Copolymer Films, Langmuir, Vol. 16, S. 407 - 415, 2000
[9] F. Burmeister et al., Mit Kapillarkräften zu Nanostrukturen, Physikalische Blätter, Vol. 36, S. 49 - 51, 2000
[10] IBA Product Guide 2001, Seite 81
[11] Roche Molecular Biochemicals, 1999 Biochemicals Catalog, S.89.

### Bezugszeichenliste

- 100: Biosensor
- 101: Photodiode
- 102: Photodiode
- 103: Schicht aus Halbleitermaterial
- 104: elektrischer Anschluss
- 105: elektrischer Anschluss
- 106: Oxidscchicht
- 107: Einheit zum Immobilisieren
- 108: Einheit zum Immobilisieren
- 109: DNA-Sondenmoleküle
- 110: DNA-Sondenmoleküle
- 111: Markierung, die Chemilumineszenzsignal erzeugt
- 112: Markierung, die Chemilumineszenzsignal erzeugt
- 113: Elektrolyt
- 114: DNA-Stränge
- 115: Nachweiskonjugat
- 116: Nachweiskonjugat
- 117: Substrat für Nachweiskonjugat
- 118: Substrat für Nachweiskonjugat
- 119: Pfeil

- 200: Biosensor
- 201: Photodiode
- 202: Photodiode
- 203: Schicht aus Halbleitermaterial
- 204: elektrischer Anschluss
- 205: elektrischer Anschluss
- 206: Oxidchicht
- 207: Einheit zum Immobilisieren
- 208: Einheit zum Immobilisieren
- 209: DNA-Sondenmoleküle
- 210: Elektrolyt
- 211: DNA-Moleküle
- 212: Markierung, die Chemilumineszenzsignal erzeugt
- 213: Nachweiskonjugat
- 214: Substrat
- 215: Pfeil

- 300: Biosensor
- 301: Photodiode
- 302: Photodiode
- 303: Schicht aus Halbleitermaterial
- 304: Oxidschicht
- 305: Schicht aus zur Immobilisierung von makromolekularen Biopolymeren nicht geeignetem Material
- 306: Nanopartikelförmige Einheiten zum Immobilisieren
- 307: DNA-Fängermolekül

- 400: Biosensor
- 401: Erste Elektrode
- 402: Zweite Elektrode
- 403: Isolatorschicht
- 404: Elektrodenanschluss
- 405: Elektrodenanschluss
- 406: DNA-Sondenmolekül
- 407: Analyt
- 408: DNA-Strang

## Patentansprüche

1. Verfahren zum Erfassen von makromolekularen Biopolymeren mittels mindestens einer Einheit zum Immobilisieren von makromolekularen Biopolymeren, welche in einem Substrat integriert ist oder auf dem Substrat aufgebracht ist,
• bei dem die mindestens eine Einheit zum Immobilisieren von makromolekularen Biopolymeren mit Fängermolekülen versehen wird, wobei die Fängermoleküle makromolekulare Biopolymere binden können,
• bei dem eine Probe mit der mindestens einen Einheit zum Immobilisieren von makromolekularen Biopolymeren in Kontakt gebracht wird, wobei die Probe die zu erfassenden makromolekularen Biopolymere enthalten kann,
• bei dem in der Probe enthaltene makromolekulare Biopolymere an den Fängermolekülen gebunden werden,
• bei dem mittels einer Markierung die Erzeugung eines Chemilumineszenzsignals angeregt wird, und
• bei dem das Chemilumineszenzsignal von einer in dem Substrat als integrierte Schaltung ausgestalteten Erfassungseinheit erfasst wird, wodurch die makromolekularen Biopolymere erfasst werden,
• bei dem das Chemilumineszenzsignal mittels einer Markierung erzeugt wird, die sich an den Fängermolekülen befindet, und
• bei dem Fängermoleküle, an die keine zu erfassenden makromolekularen biopolymere gebunden haben, entfern werden , bevor mittels der Markierung die Erzeugung des Chemilumineszenzsignals angeregt wird.

2. Verfahren nach Anspruch 1,
bei dem als makromolekulare Biopolymere Nukleinsäuren, Oligonukleotide, Proteine oder Komplexe aus Nukleinsäuren und Proteinen erfasst werden.

3. Verfahren nach einem der Ansprüche 1 oder 2,
bei dem als mindestens eine Einheit zum Immobilisieren eine Anordnung von Nanopartikeln verwendet wird.

4. Verfahren nach einem der vorhergehenden Ansprüche,
bei dem die mindestens eine Einheit zum Immobilisieren auf der Erfassungseinheit ist.

5. Verfahren nach Anspruch 4,
bei dem mehrere Einheiten zum Immobilisieren in einer regelmäßigen Anordnung auf mehreren Erfassungseinheiten aufgebracht sind.

6. Verfahren nach Anspruch 4 oder 5,
bei dem als Erfassungseinheit oder die mehreren Erfassungseinheiten eine Photodiode, eine CCD-Kamera oder eine CMOS-Kamera verwendet wird.

7. Verfahren nach einem der vorhergehenden Ansprüche,
bei dem jede Erfassungseinheit zur Erfassung des Signals individuell angesteuert wird.

8. Biosensor zum Erfassen von makromolekularen Biopolymeren mit mindestens einer Einheit zum Immobilisieren mit makromolekularen Biopolymeren, welche in einem Substrat integriert ist oder auf dem Substrat aufgebracht wird, und mit einer Erfassungseinheit,
• bei dem die mindestens eine Einheit zum Immobilisieren von makromolekularen Biopolymeren mit Fängermolekülen versehen ist, wobei die Fängermoleküle makromolekulare Biopolymere binden können, und wobei die Fängermoleküle eine Markierung aufweisen, mittels der ein Chemilumineszenzsignal erzeugt werden kann, und wobei der Biosensor derart eingerichtet ist, dass die Fängermoleküle, an die keine zu erfassenden makromolekularen Biopolymere gebunden haben, entfernbar sind, bevor mittels der Markierung die Erzeugung des Chemilumineszenzsignals angeregt wird,
• bei dem die Erfassungseinheit als eine in dem Substrat integrierte Schaltung ausgestaltet ist, und
• bei der die Erfassungseinheit derart ausgestaltet ist, dass die Erfassungseinheit makromolekulare Biopolymere, die an die Fängermoleküle gebunden haben, mittels des von der Markierung erzeugten Signals erfasst.

9. Biosensor nach Anspruch 8,
bei dem die Erfassungseinheit mindestens eine Photodiode, CCD-Kamera oder CMOS-Kamera aufweist.

10. Biosensor nach Anspruch 8 oder 9,
der mehrere Einheiten zum Immobilisieren von makromolekularen Biopolymeren in einer regelmäßigen Anordnung aufweist.

11. Biosensor nach Anspruch 9 oder 10,
bei der die Einheit zum Immobilisieren auf einer Erfassungseinheit aufgebracht ist.

## Claims

1. A method for detecting macromolecular biopolymers using a macromolecular biopolymer immobilizing unit, which is integrated in a substrate or mounted on a substrate,
• wherein the at least one macromolecular biopolymer immobilizing unit is provided with capture molecules, wherein the capture molecules are able to bind macromolecular biopolymers,
• wherein a sample is brought into contact with the at least one macromolecular biopolymer immobilizing unit, wherein the sample may contain the macromolecular biopolymers to be detected,
• wherein the macromolecular biopolymers contained in the sample are bound to the capture molecules;
• wherein the generation of a chemiluminescence signal is induced using a label, and
• wherein the chemiluminescence signal is detected using a detection unit, which is formed as an integrated circuit in the substrate, resulting in the macromolecular biopolymers being detected,
• wherein the chemiluminescence signal is generated using a label, which is located on the capture molecules, and
• wherein any capture molecules to which no macromolecular biopolymers have bound are removed, before the generation of the chemiluminescence signal is induced using the label.

2. The method as claimed in claim 1,
wherein nucleic acids, oligonucleotides, proteins or complexes composed of nucleic acids and proteins are detected as macromolecular biopolymers.

3. The method as claimed in one of claims 1 or 2,
wherein an array of nanoparticles is used as at least one immobilizing unit.

4. The method as claimed in any one of the preceding claims,
wherein the at least one immobilizing unit is on the detection unit.

5. The method as claimed in claim 4,
wherein a plurality of immobilizing units are mounted in a regular arrangement on several detection units.

6. The method as claimed in claim 4 or 5,
wherein a photodiode, a CCD camera or a CMOS camera are used as detection unit or the plurality of detection units.

7. The method as claimed in any one of the preceding claims, wherein each detection unit is actuated individually for detection of the signal.

8. A biosensor for detecting macromolecular biopolymers comprising at least one macromolecular biopolymer immobilizing unit, which is integrated in a substrate or mounted on the substrate and comprising a detection unit,
• in which the at least one macromolecular biopolymer immobilizing unit is provided with capture molecules, wherein the capture molecules are able to bind • biopolymers and wherein the capture molecules comprise a label which generates a chemiluminescence signal, and wherein the biosensor is arranged such that the capture molecules to which no macromolecular biopolymers to be detected have bound, are removable, before the creation of the chemiluminescence signal is induced using the label,
• in which the detection unit is formed as a circuit integrated in the substrate, and
• in which the detection unit is formed such that, the detection unit detects macromolecular biopolymers which have bound to the capture molecules using the signal created by the label.

9. The biosensor as claimed in claim 8,
wherein the detection unit includes at least one of a photodiode, CCD camera or CMOS camera.

10. The biosensor as claimed in any one of claims 8 or 9,
comprising several macromolecular biopolymer immobilizing units in a regular arrangement.

11. The biosensor as claimed in claim 9 or 10,
wherein the immobilizing unit is mounted on a detection unit.

## Revendications

1. Procédé de détection de biopolymères macromoléculaires au moyen d'au moins une unité d'immobilisation de biopolymères macromoléculaires, qui est intégrée dans un substrat ou qui est déposée sur le substrat,
• dans lequel on munit la au moins une unité d'immobilisation de biopolymères macromoléculaires de molécules de capture, les molécules de capture pouvant fixer des biopolymères macromoléculaires,
• dans lequel on met un échantillon en contact avec la au moins une unité d'immobilisation de biopolymères macromoléculaires, l'échantillon pouvant contenir les biopolymères macromoléculaires à détecter,
• dans lequel on fixe les biopolymères macromoléculaires contenus dans l'échantillon sur les molécules de capture,
• dans lequel on excite au moyen d'un marquage la production d'un signal de chémioluminescence, et
• dans lequel on détecte le signal de chémioluminescence par une unité de détection conformée en circuit intégré dans le substrat, en détectant ainsi les copolymères macromoléculaires,
• dans lequel on produit le signal de chémioluminescence à l'aide d'un marquage, qui se trouve sur les molécules de capture, et
• dans lequel on élimine les molécules de capture, sur lesquelles des biopolymères macromoléculaires à détecter ne se sont pas fixés, avant d'exciter au moyen du marquage la production du signal de chémioluminescence.

2. Procédé suivant la revendication 1,
dans lequel on détecte comme biopolymères macromoléculaires des acides nucléiques, des oligonucléotides, des protéines ou des complexes d'acide nucléique et de protéine.

3. Procédé suivant la revendication 1 ou 2,
dans lequel on utilise comme au moins une unité d'immobilisation un agencement de nanoparticules.

4. Procédé suivant l'une des revendications précédentes,
dans lequel la au moins une unité d'immobilisation est sur l'unité de détection.

5. Procédé suivant la revendication 4,
dans lequel plusieurs unités d'immobilisation sont détectées suivant un agencement régulier sur plusieurs unités de détection.

6. Procédé suivant la revendication 4 ou 5,
dans lequel on utilise, comme unité de détection, une photodiode, une caméra CCD ou une caméra CMOS.

7. Procédé suivant l'une des revendications précédentes,
dans lequel on commande individuellement chaque unité de détection du signal.

8. Biocapteur de détection de biopolymères macromoléculaires, comprenant au moins une unité d'immobilisation de biopolymères macromoléculaires, qui est intégrée dans un substrat ou qui est déposée sur le substrat et une unité de détection,
• dans lequel la au moins une unité d'immobilisation de biopolymères macromoléculaires est munie de molécules de capture, les molécules de capture pouvant fixer des biopolymères macromoléculaires et les molécules de capture ayant un marquage, au moyen duquel un signal de chémioluminescence peut être produit, et dans lequel le biocapteur est tel que les molécules de capture, auxquelles des biopolymères macromoléculaires à détecter ne se sont pas fixés, peuvent être éliminées avant qu'au moyen du marquage la production du signal de chémioluminescence soit excité,
• dans lequel l'unité de détection est sous la forme d'un circuit intégré dans le substrat, et
• dans lequel l'unité de détection est conformée, de manière à ce que l'unité de détection détecte au moyen du signal produit par le marquage des biopolymères macromoléculaires qui se sont fixés sur les molécules de capture.

9. Biocapteur suivant la revendication 8,
dans lequel l'unité de détection comporte au moins une photodiode, une caméra CCD ou une caméra CMOS.

10. Biocapteur suivant la revendication 8 ou 9,
qui a plusieurs unités d'immobilisation de biopolymères macromoléculaires suivant un agencement régulier.

11. Biocapteur suivant la revendication 9 ou 10,
dans lequel l'unité d'immobilisation est déposée sur une unité de détection.
